# EUROPÄISCHE PATENTSCHRIFT

(11) **EP 2 988 710 B1**
(45) Veröffentlichungstag und Bekanntmachung des Hinweises auf die Patenterteilung: **25.01.2017**
(21) Anmeldenummer: 14714590.8
(22) Anmeldetag: 21.03.2014
(51) Int. Cl.: A61F 9/007, A61F 9/008, A61N 5/06

(54) **OKULÄRE THERAPIEVORRICHTUNG**
OCULAR THERAPY DEVICE
DISPOSITIF DE TRAITEMENT OCULAIRE

(30) Priorität: 23.04.2013 DE 102013007074
(43) Veröffentlichungstag der Anmeldung: 02.03.2016
(73) Patentinhaber: Tad Bavaria GmbH, 91456 Diespeck (DE)
(72) Erfinder: REIS, Werner, 80992 München (DE); SPALTMANN, Ronald, 83224 Grassau (DE)
(74) Vertreter: Rösler, Uwe
(86) Internationale Anmeldenummer: PCT/EP2014/000798
(87) Internationale Veröffentlichungsnummer: WO 2014/173485

(56) Entgegenhaltungen:
- DE-A1-102006 030 219

## Beschreibung

### Technisches Gebiet

Die Erfindung bezieht sich auf eine okuläre Therapievorrichtung mit einer UV-Licht emittierenden Strahlungsquelle sowie einem der Strahlungsquelle nachgeordneten optischen Abbildungssystem zur Abbildung eines von der Strahlungsquelle ausgehenden Therapiestrahls in eine okuläre Abbildungsebene, mit einer der Strahlungsquelle nachgeordneten optischen Kondensoreinheit, einer Blendeneinheit, einem optischen Mittel zur Beeinflussung einer dem Therapiestrahl zuordenbaren, längs des Therapiestrahlquerschnittes orientierten räumlichen Energieverteilung sowie einem optischen Mittel zur Beeinflussung einer dem Therapiestrahl zuordenbaren Strahlform.

### Stand der Technik

Aus der DE 10 2006 030 219 A1 ist ein gattungsgemäßes Bestrahlungssystem für medizinische Anwendungen beschrieben, mit dem eine photoinduzierte Vernetzung von okulärem Gewebe, d.h. Augengewebe, mittels ultravioletter Strahlung initiiert wird. Insbesondere in Gegenwart von zusätzlich in das zu behandelnde Augengewebe eingebrachter Photosensibilisatoren vermag eine lokale Applikation von UV-Strahlung auf das zu behandelnde Auge die biomechanischen Eigenschaften des okulären Gewebes zu ändern. Mit Hilfe einer derartigen, photoinduzierten Therapie können okuläre Gewebebereiche vor allem der äußeren Augenhaut, insbesondere der Bereich der Kornea, behandelt werden. Typischerweise lassen sich Verformungen der äußeren Augenhaut, die sich krankheits- oder erblich bedingt ergeben und zumeist mit Fehlsichtigkeiten verbunden sind, durch die photoinduzierte Vernetzung von okulärem Gewebe therapieren, zumal die Behandlung eine photochemische, nicht Gewebe abtragende Stabilisierung und Veränderung der biomechanischen oder biochemischen Eigenschaften der Kornea ermöglicht.

Eine gattungsgemäße Therapievorrichtung weist typischerweise eine UV-Strahlenquelle auf, deren emittierte UV-Strahlung mit Hilfe eines aus wenigstens zwei Linsen bestehenden optischen Systems auf eine okuläre Abbildungsebene abgebildet wird, in die der zu therapierende Augenabschnitt eines zu behandelnden Patienten, bspw. die Kornea des Patientenauges, zu positionieren ist. Zur Beeinflussung von Strahlgröße sowie auch Energieverteilung längs des Strahlquerschnittes des in die okulären Abbildungsebene abgebildeten Therapiestrahls sind zudem längs des Therapiestrahlenganges eine den Therapiestrahl geometrisch begrenzende Blende sowie ein auf die Energieverteilung des Therapiestrahl Einfluss nehmendes optisches Element mit diffraktiver oder holographischer Wirkung eingebracht.

Der EP 2 380 535 A1 ist eine Vorrichtung für die Vernetzung von okulärem Gewebe mit elektromagnetischer Strahlung zu entnehmen, bei der längs des Therapiestrahlenganges ein optisches Mittel zum Einstellen einer inhomogenen Verteilung der Strahlungsstromdichte des in die okuläre Abbildungsebene abgebildeten Therapiestrahls eingebracht ist. Insbesondere vermag das optische Mittel die Wirktiefe der elektromagnetischen Strahlung innerhalb des zu therapierenden, okulären Gewebes, das sich in der okulären Abbildungsebene befindet, in gewünschter Weise unterschiedlich einzustellen. Bspw. eignet sich hierzu eine vom Therapiestrahl durchsetzte Absorberplatte, die längs des Therapiestrahlquerschnittes über unterschiedliche Absorptionsbereiche verfügt.

Aus DE 10 2010 020 194 A1 sind ein Verfahren und eine Vorrichtung zur Stabilisierung der Augenhornhaut bekannt, bei denen die Hornhaut sukzessiv an verschiedenen Stellen lokal derart bestrahlt wird, dass an den bestrahlten Stellen Kollagenfasern miteinander vernetzen. Zu Bestrahlung an verschiedenen Stellen ist der Behandlungsstrahlengang optisch so ausgebildet, dass der Laserstrahl zu einem Zeitpunkt nur einen lokalen Teil der Hornhaut bestrahlt, nicht aber die gesamte Hornhaut.

Bislang bekannte, gattungsgemäße Therapievorrichtungen verfügen über einen komplexen und zumeist kostenaufwendigen aparativen Aufbau, den es von einem zur Durchführung augenchirurgischer Therapiemaßnahmen befähigten Operateur exakt gegenüber eines räumlich fixierten, zu behandelnden Patientenauges zu justieren gilt.

### Darstellung der Erfindung

Der Erfindung liegt die Aufgabe zugrunde, eine gattungsgemäße okuläre Therapievorrichtung mit einer UV-Licht emittierenden Strahlungsquelle sowie einem der Strahlungsquelle nachgeordneten optischen Abbildungssystem zur Abbildung eines von der Strahlungsquelle ausgehenden Therapiestrahls in eine okuläre Abbildungsebene, mit einer der Strahlungsquelle nachgeordneten optischen Kondensoreinheit, einer Blendeneinheit, einem optischen Mittel zur Beeinflussung einer dem Therapiestrahl zuordenbaren, längs des Therapiestrahlquerschnittes orientierten räumlichen Energieverteilung sowie einem optischen Mittel zur Beeinflussung einer dem Therapiestrahl zuordenbaren Strahlform derart weiterzubilden, so dass die Kosten zur Realisierung der Therapievorrichtung signifikant gesenkt werden können, ohne dabei Nachteile hinsichtlich der Strahleigenschaften des in die okuläre Abbildungsebene abgebildeten Therapiestrahls in Kauf nehmen zu müssen. Vielmehr gilt es überdies die Bedienungsfreundlichkeit der Therapievorrichtung für den behandelnden Augenarzt zu verbessern sowie die therapeutisch wirksamen Strahleigenschaften möglichst optimiert an die Augengeometrie des zu behandelnden Auges anzupassen.

Die Lösung der der Erfindung zugrunde liegenden Aufgabe ist im Anspruch 1 angegeben. Den lösungsgemäßen Gedanken in vorteilhafter Weise weiterbildende Merkmale sind Gegenstand der Unteransprüche sowie der weiteren Beschreibung insbesondere unter Bezugnahme auf die Ausführungsbeispiele zu entnehmen.

Die lösungsgemäße okuläre Therapievorrichtung gemäß den Merkmalen des Oberbegriffes des Anspruches 1 zeichnet sich dadurch aus, dass das Mittel zur Beeinflussung der dem Therapiestrahl zuordenbaren Energieverteilung eine mittels Ätztechnik bearbeitete Blende ist, die als sog. Ätzblende eine kostengünstig herstellbare Komponente darstellt. Alternativ zur Ätzblende eignet sich ebenso eine Strichplatte, die eine transparente Platte mit wenigstens einem zumindest einseitig bedruckten Liniengitter vorsieht, dessen Linien für das UV-Licht der Strahlungsquelle intransparent sind.

Ferner ist das optische Mittel zur Beeinflussung der dem Therapiestrahl zuordenbaren Strahlform in Form wenigstens einer asphärischen optischen Linse ausgebildet, durch die der Therapiestrahl auf die räumlich gekrümmte Form der Kornea eines zu behandelnden Patientenauges scharf abgebildet wird, d. h. die okuläre Abbildungsebene nimmt die Form einer gekrümmten Fokalfläche an, die vorzugsweise an die Krümmung der natürlichen Augenkrümmung angepasst ist. Aus diesem Grunde wird im Nachfolgenden der Begriff der "okulären Abbildungsebene" als räumlich gekrümmte Fokalfläche verstanden, längs der die Vielzahl der einen scharf auf die okuläre Abbildungsebene abgebildeten Strahlquerschnitt zusammensetzenden Strahlfokuspunkte zu liegen kommt.

Zu Zwecken einer erleichterten und zugleich exakten Positionierung der okulären Therapievorrichtung gegenüber eines zu behandelnden Patientenauges, d. h. zu Zwecken der Zentrierung sowie Fokussierung des Therapiestrahls relativ zu einem zu behandelnden und räumlich fixierten Auge, sind räumlich um den auf die okuläre Abbildungsebene gerichteten Therapiestrahl wenigstens drei räumlich getrennte Ziellichtstrahlen angeordnet, längs deren einzelner Strahlengänge jeweils eine stenopäische Blende eingebracht ist, dessen Blendenloch durch eine optische Abbildungseinheit in die okuläre Abbildungsebene abbildbar ist, so dass die in der okulären Abbildungsebene abgebildeten, wenigstens drei Ziellichtstrahlen gleich verteilt auf einer Kreislinie liegen, deren Kreisdurchmesser zwischen 3 und 15 Millimeter, vorzugsweise zwischen 5 und 8, besonders vorzugsweise 6 Millimeter misst. Die Wellenlänge der einzelnen Ziellichtstrahlen ist derart gewählt, so dass ein zu behandelnder Augenarzt die an der Hornhautoberfläche durch die Ziellichtstrahlen erzeugten Reflexbilder möglichst kontrastreich zu erkennen vermag und diese somit dem Augenarzt als exakte Justierhilfen dienen. Der Therapiestrahl, dessen im ultravioletten Spektralbereich liegende Wellenlänge dem Augenarzt unsichtbar verborgen bleibt, ist gegenüber dem zu behandelnden Auge korrekt justiert bzw. positioniert, sobald die Blendenlöcher der Ziellichtstrahlen scharf und konzentrisch um die Pupille des zu behandelnden Auges abgebildet werden.

Ferner gilt es dafür Sorge zu tragen, dass die Blickrichtung des zu behandelnden Patientenauges während der Untersuchung möglichst unverändert bleibt. Als Justierhilfe für den Patienten ist hierzu längs des auf die okuläre Abbildungsebene gerichteten Therapiestrahls zusätzlich ein Fixierstrahl angeordnet, längs dem eine stenopäische Blende eingebracht ist, dessen Blendenloch über eine Abbildungsoptik in eine der okulären Abbildungsebene in Strahlrichtung nachgelagerte Abbildungsebene scharf abbildbar ist. Hierbei gilt es die hierzu vorzusehende Abbildungsoptik geeignet zu wählen, so dass das Abbild des Blendenloches längs des Fixierlichtstrahls vom zu behandelnden Auge des Patienten scharf gesehen wird. In Folge dessen gilt es das Blendenloch somit scharf auf die Netzhaut des zu behandelnden Auges des Patienten abzubilden. In einer bevorzugten Ausführungsform dient als die den Fixierlichtstrahl abbildende Abbildungsoptik die wenigstens eine asphärische optische Linse selbst, mit der die Strahlform des Therapiestrahls an die Augenkrümmung des zu behandelnden Auges angepasst und optisch scharf abgebildet wird.

Durch die Verwendung einer mittels kostengünstiger Ätztechnik herstellbaren Blende, die über für den Therapiestrahl durchlässige Blendenbereich verfügt, deren Anzahl, Form und Größe je nach erwünschter Energieverteilung längs des Therapiestrahlquerschnittes beliebig vorgebbar sind, steht eine kostengünstige Komponente zur Verfügung, die als auswechselbares Einsetzmodul in den Strahlengang des Therapiestrahls einsetzbar ist und in unterschiedlichen Ausbildungsformen nach Art eines Baukastenprinzips bevorratet werden kann, um die Therapievorrichtung individuell an Patientenspezifische Therapiebedürfnisse anzupassen.

In gleichem Masse lässt sich anstelle der Ätzplatte eine Strichplatte mit einem geeignet designten Strichmuster einsetzen, dessen Strichmusterlinien feinste Strukturen darstellen.

In gleicher Weise stellt die wenigstens eine asphärische optische Linse zur Strahlformung des auf die okuläre Abbildungsebene abbildbaren Therapiestrahls eine kostengünstige Maßnahme dar, mit der es möglich ist, den in seiner Energieverteilung individuell vorgegebenen Therapiestrahl scharf auf die räumlich gekrümmte und an die Oberflächenform des Auges angepasste okuläre Abbildungsebene oder besser gesagt okuläre Fokalfläche abzubilden. Durch die vorstehend genannten beiden Maßnahmen können entscheidende Kostenreduzierungen erzielt werden.

Durch das zusätzliche Vorsehen wenigstens dreier, räumlich um den auf die okuläre Abbildungsebene gerichteten Therapiestrahl, getrennt angeordneter Ziellichtstrahlen sowie den längs des Therapiestrahls verlaufenden Fixierlichtstrahls wird die Handhabung der lösungsgemäß ausgebildeten Therapievorrichtung für den Augenarzt zum Zwecke einer exakten Justierung der Therapievorrichtung relativ zu dem zu behandelnden Auge signifikant verbessert. Durch die unabhängig vom Seheindruck des zu behandelnden Patienten durch den Augenarzt vornehmbare Positionierung der Therapievorrichtung gegenüber dem zu behandelnden Auge, ist eine Kommunikation zwischen Patient und Augenarzt nicht erforderlich, so dass die Behandlung von fremdsprachigen oder zur Sprache nicht oder noch nicht befähigten Patienten, bspw. Babys, Kleinkinder oder Tieren, problemlos möglich ist.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematisierte Darstellung des Strahlengangs sowie der hierfür erforderlichen optischen Komponenten zur Realisierung der lösungsgemäß ausgebildeten Therapievorrichtung,
- Fig. 2a, b: Detaildarstellung zur Illustration der Komponenten zur Realisierung der Ziellichtstrahlen,
- Fig. 3a, b, c, d: alternative Ausführungsformen zur Realisierung einer Ätzblende, sowie einer Strichplatte,
- Fig. 4: Detaildarstellung zur Illustration der Erzeugung und Abbildung des Fixierlichtstrahls auf die Netzhaut eines zu behandelnden Auges,
- Fig. 5a, b: Gegenüberstellung des Strahlenverlaufes zwischen einer optischen Linse mit und ohne Asphäre und
- Fig. 6: schematisierte Darstellung einer Komponentendarstellung für eine lösungsgemäß ausgebildete Therapievorrichtung mit Einkopplung eines weiteren Strahlenganges, bspw. zur Untersuchung des Augenhintergrundes.

### Wege zur Ausführung der Erfindung, gewerbliche Verwendbarkeit

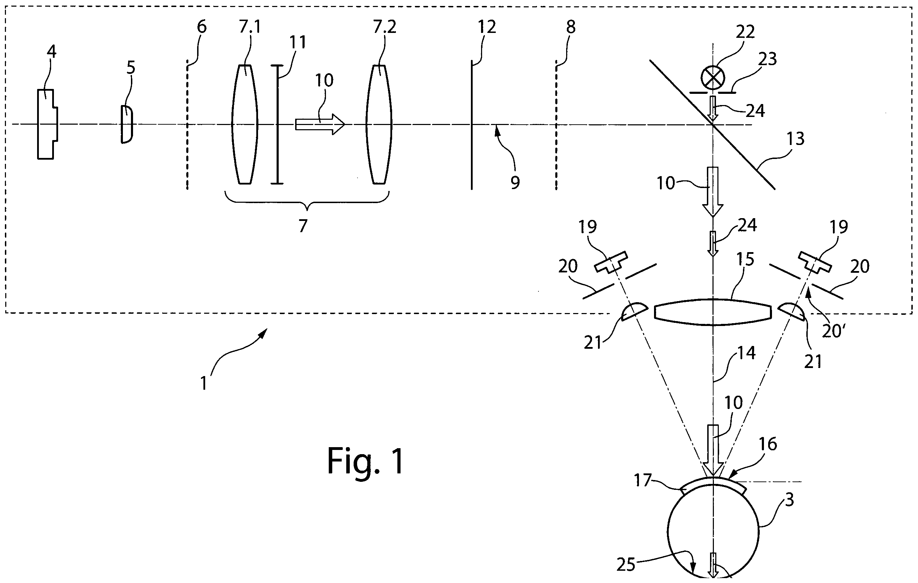

Figur 1 zeigt in schematisierter Darstellung den Komponentenaufbau einer lösungsgemäß ausgebildeten Therapievorrichtung 1, die in einem manuell handhabbaren Gehäuse 2 integriert sind, das gegenüber einem zu behandelnden Auge 3 zu positionieren ist. Die Therapievorrichtung 1 umfasst eine Strahlungsquelle 4, die UV-Strahlung emittiert. Vorzugsweise eignen sich LED-Lichtquellen oder Laser. Das aus der Strahlungsquelle 4 austretende UV-Licht wird von einer Kondensoreinheit 5 zur homogenen Ausleuchtung einer ersten Zwischenbildebene 6 gebündelt und in diese abgebildet, die von einer aus zwei Linsen 7.1 und 7.2 zusammengesetzten Beleuchtungsoptik 7 in eine zweite Zwischenbildebene 8 abgebildet wird. Zur lateral räumlichen Begrenzung des sich längs der durch die Beleuchtungsoptik festgelegten, ersten optischen Achse 9 ausbreitenden Therapiestrahls 10 dient eine unmittelbar nach der ersten Linse 7.1 der Beleuchtungsoptik 7 angebrachte Irisblende 11, deren Blendendurchmesser variabel einstellbar ist. Die Wahl des Blendendurchmessers kann in Abhängigkeit der jeweils benötigten therapeutisch wirksamen Strahldurchmessergröße am Ort des zu behandelnden Auges mit Hilfe einer geeignet ausgebildeten Stelleinheit vorgenommen werden.

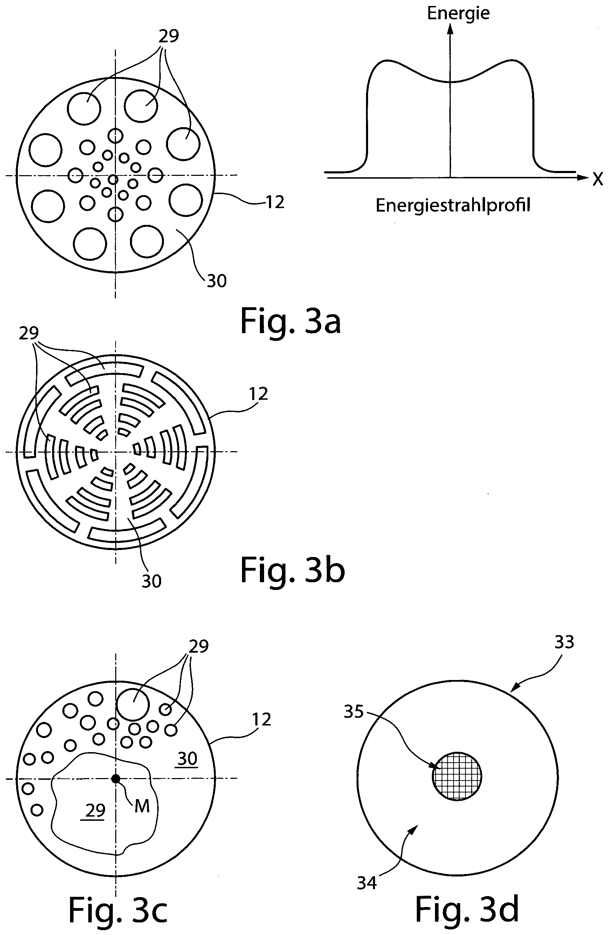

Ferner befindet sich längs der ersten optischen Achse 9 der Beleuchtungsoptik 7 im Strahlengang nachgeordnet, außerhalb der zweiten Zwischenbildebene 8 eine Ätzblende 12, durch deren flächige Verteilung der für den Therapiestrahl 10 durchlässigen Blendenbereiche sowie deren Form und Größe die Energieverteilung des Therapiestrahls 10 längs dessen Therapiestrahlquerschnittes geeignet vorgebbar ist. Einzelheiten zur möglichen Ausbildung einer derartigen Ätzblende folgen im Weiteren unter Bezugnahme auf die Figur 3. Anstelle der Ätzblende 12 lässt sich ebenso eine Strichplatte 33 einsetzen, zu deren mögliche Ausbildung auf Figur 3d verwiesen wird.

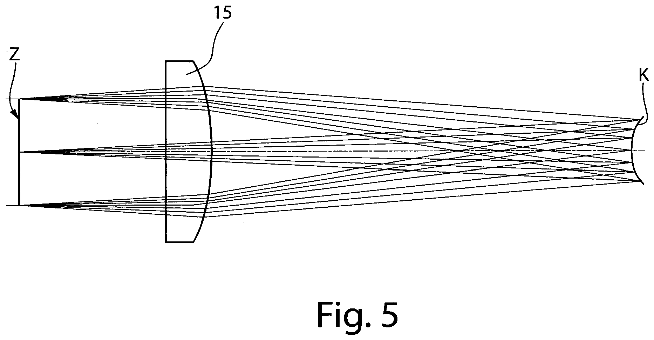

Eine im Strahlengang des Therapiestrahls 10 angebrachte optische Umlenkeinheit 13, die vorzugsweise in Form eines Teilerspiegels ausgebildet ist, sorgt für eine vorzugsweise um 90° orientierte Umlenkung des Therapiestrahls 10 aus der ersten optischen Achse 9 in eine zweite optische Achse 14, längs der der Umlenkeinheit 13 nachgeordnet eine asphärische Linse 15 angebracht ist. Die asphärische Linse 15 ist derart angeordnet und ausgebildet, dass sie den Therapiestrahl 10 scharf auf eine okuläre Abbildungsebene 16 zu fokussieren vermag, wobei die okuläre Abbildungsebene 16 einer gekrümmten Fokalfläche entspricht, die der gekrümmten Hornhautoberfläche 17 des zu behandelnden Auges 3 entspricht. Durch die asphärische Linse 15 wird somit das Strahlprofil des auf das zu behandelnde Auge auftreffenden Therapiestrahls 10 derart geformt, so dass der Therapiestrahl 10 in seiner gesamten Querschnittsfläche exakt fokussiert auf die natürlich gekrümmten Hornhautoberfläche abgebildet wird. Zur Illustration dieser Strahlformung sei an dieser Stelle auf die Figur 5 verwiesen, in der die dort dargestellte Kreislinie K der natürlich gekrümmten Hornhautoberfläche 17 entspricht. Durch die asphärische Linse 15 wird die Fokalebene der optischen Abbildung exakt längs der gekrümmten Hornhautoberfläche 17 verformt, so dass auf der gesamten Hornhautoberfläche eine scharfe Abbildung des Therapiestrahls 10 gewährleistet ist, siehe Figur 5. In diesem Fall bildet eine asphärische Linse 15 einen scharf in eine Zwischenbildebene Z abgebildeten Strahl auf eine gekrümmte Fokalebene scharf ab, die im Fall der Anmeldegegenstandes der okulären, "gekrümmten" Abbildungsebene 16 entspricht. Deutlich zu erkennen ist, dass die Vielzahl der den Strahlfokus in seiner gesamten lateralen Strahlquerschnittsausdehnung zusammensetzenden Strahlfokuspunkte längs der kreisförmig gekrümmten Fokalfläche K liegen.

Zusätzlich sieht die lösungsgemäß ausgebildete Therapievorrichtung 1 drei gleich verteilt um die zweite optische Achse 14 angeordnete Ziellichtstrahlen 18 vor, von denen aus zeichnerischen Gründen in Figur 1 lediglich zwei dargestellt sind, die es dem zu behandelnden Augenarzt erleichtern, die Therapievorrichtung 1 relativ zu einem räumlich fixierten, zu behandelnden Patientenauge 3 exakt zu positionieren.

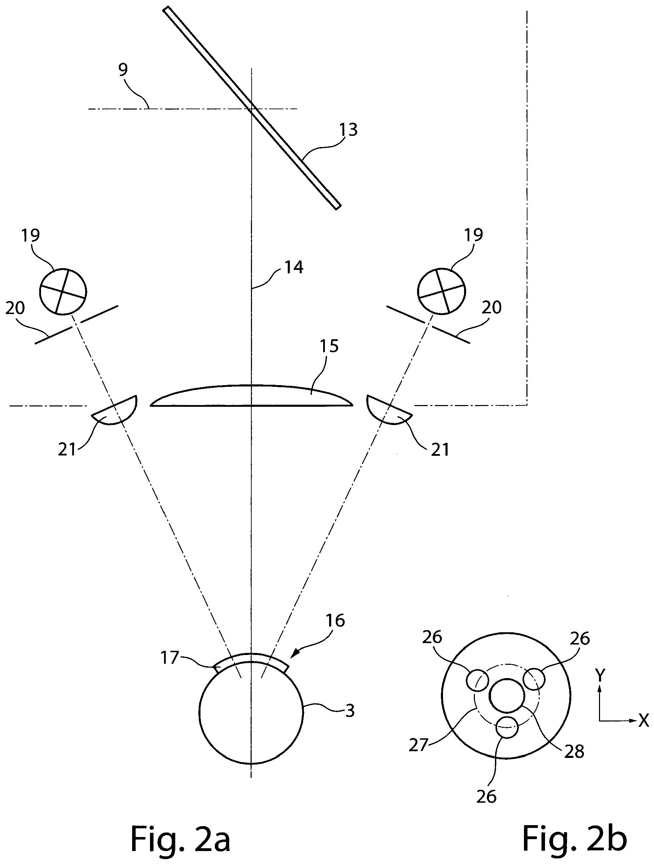

Zur Erzeugung der wenigstens drei Ziellichtstrahlen 18 dient jeweils eine Lichtquelle 19, deren Wellenlänge im sichtbaren Spektralbereich liegt und derart gewählt ist, so dass sie in Reflexion an der Iris möglichst kontrastreich in Erscheinung tritt. Im Strahlengang jedes einzelnen auf das Auge 3 gerichteten Ziellichtstrahls 18 befindet sich eine stenopäische Blende 19, die über ein kreisrundes Blendenloch 19', typischerweise mit einem Lochdurchmesser von 0,5 bis 2 mm, verfügt. Das Blendenloch 19' wird über eine optische Abbildungseinheit 20, vorzugsweise ebenfalls in Form einer asphärischen Linse, scharf auf die Iris abgebildet, so dass die in der okulären Abbildungsebene 16 abgebildeten, wenigstens drei Ziellichtstrahlen 18 gleich verteilt auf einer Kreislinie liegen, deren Kreisdurchmesser zwischen 3 und 15 Millimeter, vorzugsweise 5 bis 8, besonders vorzugsweise 6 Millimeter misst, wobei der Kreismittelpunkt der geometrischen Mitte des in die okuläre Abbildungsebene scharf abgebildeten Therapiestrahls 10 entspricht. Weitere Einzelheiten werden in diesem Zusammenhang im Weiteren mit Figur 2 erläutert.

Ferner weist die lösungsgemäß ausgebildete Therapievorrichtung 1 eine weitere Lichtquelle 22 auf, deren Licht gleichfalls im sichtbaren Spektralbereich liegt, sich jedoch in der Wellenlänge von den Ziellichtstrahlen 18 im Farbeindruck deutlich unterscheidet. Die Lichtquelle 22 ist rückseitig zur optischen Umlenkeinheit 13 angeordnet. Die optische Umlenkeinheit 13 ist vorzugsweise als teildurchlässiger Spiegel ausgebildet, der einerseits den Therapiestrahl 10 möglichst verlustfrei umlenkt, andererseits jedoch das von der Lichtquelle 22 emittierte Licht nahezu verlustfrei durchlässt. Der Lichtquelle 22 unmittelbar nachgeordnet ist eine stenopäische Blende 23, die einen sog. Fixierlichtstrahl 24 formt, der längs der zweiten optischen Achse 14 im weiteren die asphärische Linse 15 durchsetzt, durch die der Fixierlichtstrahl 24 derart auf das Auge gerichtet wird, so dass der Fixierlichtstrahl 24 scharf auf der Retina 25 des zu behandelnden Auges 3 abgebildet wird. Der Fixierlichtstrahl 24 hilft insbesondere dem Patienten die Blickrichtung des zu behandelnden Auges 3 während der therapeutischen Maßnahme unverändert zu belassen.

Figur 2a stellt eine vergrößerte schematische Seitenansicht des optischen Aufbaus zur Erzeugung der Ziellichtstrahlen 18 dar. Von den wenigstens drei Ziellichtstrahlen 18 sind in Figur 2a zwei Ziellichtstrahlen 18 gezeigt, zu deren Erzeugung jeweils eine LED-Lichtquelle 19 innerhalb der Therapievorrichtung 1 angebracht ist. Die LED-Lichtquelle 19 emittiert Licht im sichtbaren Spektralbereich, vorzugsweise grünes Licht, das durch eine in unmittelbarer Nähe zur Lichtquelle 19 angeordnete stenopäische Blende 20 hindurchtritt, die über ein Blendenloch 20' mit einem Lochdurchmesser von vorzugsweise 1 mm verfügt. Der durch die stenopäische Blende 20 hindurchtretende Ziellichtstrahl 18 gelangt nachfolgend auf eine optische Abbildungseinheit 20, die als asphärische Linse ausgebildet ist, die das Blendenloch 20' scharf in die okuläre Abbildungsebene 16 abbildet.

Die den jeweiligen Strahlengängen der wenigstens drei Ziellichtstrahlen 18 zugeordneten asphärischen Linsen 20 sind gleich verteilt um die und unmittelbar neben der asphärischen Linse 15 angeordnet, die der Abbildung des Therapiestrahls 10 auf die okuläre Abbildungsebene 16 dient. Somit befinden sich die Abbilder der Blendenlöcher 20' in der okulären Abbildungsebene 16 gleich verteilt längs einer Kreislinie, deren Kreisdurchmesser typischerweise 6 mm beträgt. In Figur 2b sind die Blendenlochabbilder 26 längs der Kreislinie 27 illustriert. Sobald ein zu behandelnder Augenarzt die Vorrichtung gegenüber dem zu behandelnden Auge 3 derart justiert, so dass sich das in Figur 2b illustrierte Reflexbild an der Augenoberfläche ergibt, d. h. die Reflexbilder 26 der Blendenlöcher 20' befinden sich konzentrisch zur Pupille 28 des zu behandelnden Auges 3, ist die Konzentrizität des Therapiestrahls 10 in x-y-Richtung relativ zum Auge sichergestellt. Sobald alle drei Reflexbilder 26 für den zu behandelnden Arzt wahrnehmbar scharf auf der Iris des zu behandelnden Auges 3 abgebildet sind, ist überdies sichergestellt, dass der korrekte Abstand h zwischen Therapievorrichtung 1 und Auge 3 eingestellt ist.

Die Figuren 3a, b und c stellen unterschiedlich ausgeführte Ätzblenden 12 dar, die jeweils über für den Therapiestrahl 10 durchlässige Blendenbereiche 29 verfügen, die jeweils von einem für den Therapiestrahl 10 undurchlässigen übrigen Blendenbereich 30 umgeben sind. Die räumliche Verteilung sowie die Ausbildung von Form und Größe der Vielzahl einzelner für den Therapiestrahl durchlässigen Blendenbereiche 29 sind grundsätzlich beliebig wählbar und nach Art und Beschaffenheit des jeweils zu therapierenden Auges auszuwählen. Gilt es bspw. eine Energieverteilung längs des Strahlquerschnittes des Therapiestrahls zu erzeugen, die dem in Figur 3a dargestellten Energieprofil entspricht, das weitgehend durch ein rechteckförmiges Energieprofil charakterisiert ist, mit einer zentralen Delle in der Energieverteilung, so sind die für den Therapiestrahl durchlässigen Blendenbereiche 29 in Form und Größe gemäß der in Figur 3a dargestellten Ätzblende 12 vorzunehmen. Die durchlässigen Blendenbereiche 29 können kreisförmig, elliptisch, rechteckförmig, segmentartig oder grundsätzlich beliebig geformte einstückig zusammenhängende Flächenbereiche darstellen. In Figur 3b stellen die durchlässigen Blendenbereiche 29 segmentartige Flächenformen dar, in Figur 3c ist ein Ausführungsbeispiel gezeigt, das eine asymmetrische Energieverteilung längs des Strahlquerschnittes aufweist, zumal der größte durchlässige Blendenbereich 29 azentrisch zum geometrischen Blendenmittelpunkt M der Ätzblende 12 positioniert ist. Ferner ist eine Vielzahl kleinerer Blendenbereiche azentrisch und asymmetrisch relativ zum Blendenmittelpunkt M angeordnet. Figur 3d zeigt eine Strichplatte 33, die zentrisch ein Liniengitter mit einer Vielzahl von sich kreuzenden Linien, die jeweils undurchlässige Plattenbereiche 35 für den Therapiestrahl darstellen, aufweist. Im Bereich des Liniengitters erfährt der Therapiestrahl eine Schwächung, wodurch der durch die Strichplatte 33 hindurchtretende Therapiestrahl eine Intensitätsschwächung im Strahlzentrum erfährt, wohingegen der im Bereich der durchlässigen Plattenbereiche 34 durchtretende Strahl keine Schwächung erfährt.

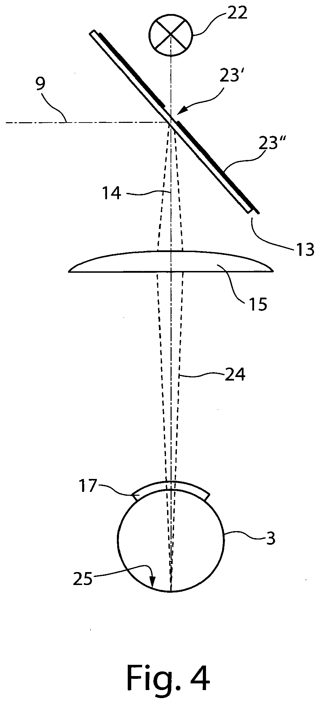

Figur 4 stellt eine Detaildarstellung zur Erzeugung des Fixierlichtstrahls 24 dar, der für den zu behandelnden Patienten dazu dient, das jeweils mit dem Therapiestrahl beaufschlagte Auge 3 während der Behandlung möglichst unbewegt und exakt ausgerichtet zum Fixierlichtstrahl 24 zu halten. Hierzu befindet sich rückseitig zur optischen Umlenkeinheit 13 eine LED-Lichtquelle 22 die Licht im sichtbaren Spektralbereich emittiert. Die optische Umlenkeinheit 13 ist grundsätzlich als für den nicht in Figur 4 eingezeichneten Therapiestrahl als Vollspiegel ausgebildet, verfügt jedoch zumindest im zentralen Bereich über eine optische Teilerfunktion, die es ermöglicht Licht der LED-Lichtquelle 22 durch die optische Umlenkeinheit 13 in Transmission passieren lassen. Hierzu befindet sich rückseitig zum Umlenkspiegel 13 eine stenopäische Blende mit einer Blendenöffnung von ca. 1 mm Lochdurchmesser, die den Fixierstrahl stark in Richtung der zweiten optischen Achse passieren lässt. Die stenopäische Blende 23 kann in Form einer rückseitig an der Umlenkeinheit 13 aufgebrachten Materialschicht 23" realisiert sein, die mit Ausnahme der Blendenöffnung für das von der LED-Lichtquelle 22 emittierte Licht undurchlässig ist. Die im Strahlengang nachfolgende asphärische Linse 15 bildet in Verbindung mit den optischen Abbildungseigenschaften des Auges 3 selbst die Blendenöffnung 23' scharf auf der Retina, bzw. der Netzhaut 25 des Auges 3 ab.

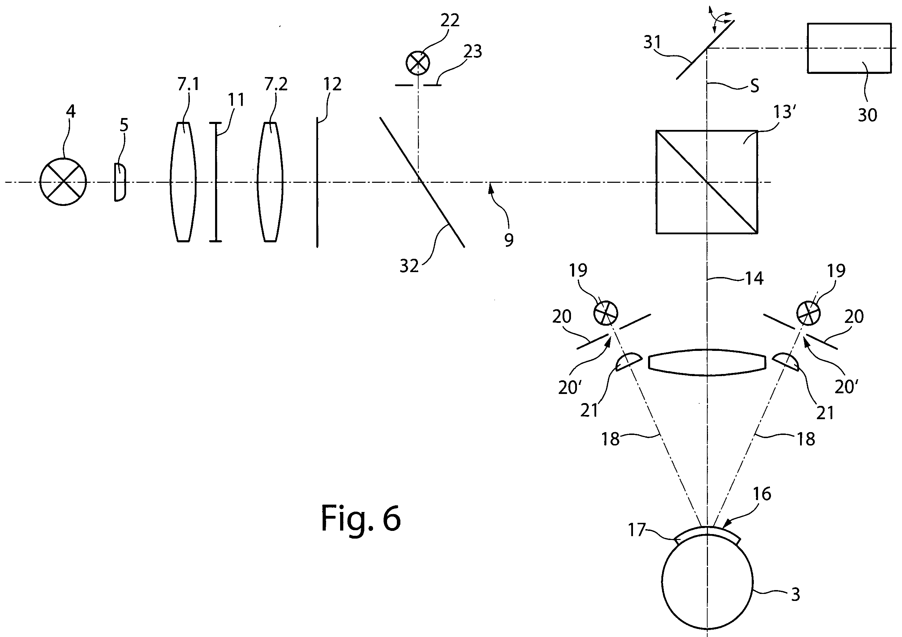

In Figur 6 ist eine alternative Ausführungsform zur Realisierung der lösungsgemäßen Therapievorrichtung dargestellt, mit der es möglich ist, neben dem Therapiestrahl 10 sowie Fixierlichtstrahl 24 einen weiteren Strahlengang längs der zweiten optischen Achse 14 einzukoppeln, mit dem der Strahlengang bspw. weitere Diagnosegeräte zur Augenuntersuchung einkoppelbar sind. Zur Vermeidung von einer wiederholten Erläuterung bereits beschriebener Komponenten werden die in Figur 6 dargestellten Komponenten mit den bereits eingeführten und erläuterten Bezugszeichen versehen. Im Unterschied zu dem in Figur 1 illustrierten Geräteaufbau ist anstelle einer als Teilerspiegel ausgebildeten optischen Umlenkeinheit 13 ein prismatischer Teilerwürfel 13' angebracht, der zum einen den Therapiestrahl 10 in gleicher Weise aus der ersten optischen Achse 9 in die zweite optische Achse 14 umzulenken vermag, andererseits jedoch einen weiteren Strahlengang S längs der zweiten optischen Achse 14 zur Augenuntersuchung einzukoppeln vermag. So rührt der weitere Strahlengang S bspw. von einer Anordnung zur Durchführung einer optischen Kohärenztomografie, kurz OCT 30, her, dessen Beleuchtungs- und Beobachtungsstrahlengang über eine den Strahlengang S umlenkende Scanneroptik 31 zur Untersuchung des Augenhintergrundes umgelenkt wird.

Die Einkopplung des Fixierlichtstrahls 24, der zur Blickrichtungskontrolle für den zu behandelnden Patienten dient, erfolgt über einen Teilerspiegel 32, der den Fixierlichtstrahl 24 zunächst längs der ersten optischen Achse 9 in Überlagerung mit dem Therapiestrahl 10 einkoppelt, der gemeinsam mit dem Therapiestrahl 10 innerhalb des Teilerwürfels 13' in Richtung der zweiten optischen Achse 14 auf das zu untersuchende Auge gerichtet wird.

### Bezugszeichenliste

- 1: Therapievorrichtung
- 2: Gehäuse
- 3: Auge
- 4: Strahlungsquelle
- 5: Kondensoreinheit
- 6: Erste Zwischenbildebene
- 7: Beleuchtungsoptik
- 7.1: Linse
- 7.2: Linse
- 8: Zweite Zwischenbildebene
- 9: Erste optische Achse
- 10: Therapiestrahl
- 11: Irisblende
- 12: Ätzblende
- 13: Optische Umlenkeinheit
- 13': Teilerwürfel
- 14: Zweite optische Achse
- 15: Asphärische Linse
- 16: Okuläre Abbildungsebene
- 17: Hornhautoberfläche
- 18: Ziellichtstrahl
- 20: Stenopäische Blende
- 20': Blendenloch
- 21: Optische Abbildungseinheit, asphärische Linse
- 22: Lichtquelle
- 23: Stenopäische Blende
- 23': Blendenöffnung
- 23": Materialschicht mit Blendenöffnung
- 24: Fixierlichtstrahl
- 25: Retina
- 26: Reflexbild des Blendenloches 20'
- 27: Kreislinie
- 28: Pupille
- 30: Ophthalmoskop
- 31: Scanneroptik
- 32: Teilerspiegel
- 33: Strichplatte
- 34: durchlässige Plattenbereiche
- 35: undurchlässige Plattenbereiche

## Patentansprüche

1. Okuläre Therapievorrichtung mit einer UV-Licht emittierenden Strahlungsquelle (4) sowie einem der Strahlungsquelle (4) nachgeordneten optischen Abbildungssystem zur Abbildung eines von der Strahlungsquelle (4) ausgehenden Therapiestrahls(10) in eine okuläre Abbildungsebene (16), mit einer der Strahlungsquelle (4) nachgeordneten optischen Kondensoreinheit (5), einer Blendeneinheit (11), einem optischen Mittel zur Beeinflussung einer dem Therapiestrahl zuordenbaren, längs des Therapiestrahlquerschnittes orientierten räumlichen Energieverteilung (12) sowie einem optischen Mittel zur Beeinflussung einer dem Therapiestrahl zuordenbaren Strahlform (15),
**dadurch gekennzeichnet, dass** das Mittel zur Beeinflussung der dem Therapiestrahl zuordenbaren Energieverteilung eine mittels Ätztechnik bearbeitete Blende ist, eine so genannte Ätzblende (12) oder eine mittels Drucktechnik bedruckte Strichplatte (33) ist,
dass das optische Mittel zur Beeinflussung der dem Therapiestrahl zuordenbaren Strahlform wenigstens eine asphärische optische Linse (15) umfasst,
dass räumlich um den auf die okuläre Abbildungsebene (16) gerichteten Therapiestrahl (10) wenigstens drei räumlich getrennte Ziellichtstrahlen (18) angeordnet sind, längs denen jeweils eine stenopäische Blende (20) eingebracht ist, dessen Blendenloch (20') jeweils durch eine optische Abbildungseinheit (21) in die okuläre Abbildungsebene (16) abbildbar ist, so dass die in der okulären Abbildungsebene (16) abgebildeten, wenigstens drei Ziellichtstrahlen (18) gleichverteilt auf einer Kreislinie (K) liegen, deren Kreisdurchmesser zwischen 3 und 15 mm misst,
und
dass längs des auf die okuläre Abbildungsebene (16) gerichteten Therapiestrahls (10) ein auf die okuläre Abbildungsebene (16) orientierter Fixierlichtstrahl (24) angeordnet ist, längs dem eine stenopäische Blende (23) eingebracht ist, dessen Blendenloch (23') über eine Abbildungsoptik in eine der okulären Abbildungsebene (16) in Strahlrichtung nachgelagerten Abbildungsebene abbildbar ist.

2. Therapievorrichtung nach Anspruch 1,
**dadurch gekennzeichnet, dass** das optische Abbildungssystem
- die Kondensoreinheit (5), die den aus der Strahlenquelle (4) austretenden Therapiestrahl (10) längs einer ersten optischen Achse (9) in eine erste Zwischenbildebene (6) abbildet,
- eine aus wenigstens zwei optischen Linsen (7.1, 7.2) bestehende Beleuchtungsoptik (7), die die erste Zwischenbildebene (6) in eine zweite Zwischenbildebene (8) abbildet, sowie
- die wenigstens eine asphärische optische Linse (15), die die zweite Abbildungsebene (8) in die okuläre Abbildungsebene (16) abbildet,
umfasst, und
dass zwischen der Beleuchtungsoptik (7) und der wenigstens einen asphärischen optischen Linse (15) eine optische Umlenkeinheit (13) eingebracht ist, die den längs der ersten optischen Achse (9) orientierten Therapiestrahl (10) in eine auf die okuläre Abbildungsebene (16) gerichtete zweite optische Achse (14) umlenkt.

3. Therapievorrichtung nach Anspruch 1 oder 2,
**dadurch gekennzeichnet, dass** zwischen den wenigstens zwei optischen Linsen (7.1, 7.2) der Beleuchtungsoptik (7) eine Irisblende (11) und zwischen der Beleuchtungsoptik (7) und der wenigstens einen asphärischen Linse (15) die Ätzblende (12) oder die bedruckte Strichplatte (33) außerhalb der zweiten Zwischenbildebene (8) angeordnet sind.

4. Therapievorrichtung nach einem der Ansprüche 1 bis 3,
**dadurch gekennzeichnet, dass** die Ätzblende (12) eine Vielzahl für den Therapiestrahl (10) durchlässige Blendenbereiche (29) besitzt, die jeweils von einem für den Therapiestrahl (10) undurchlässigen Blendenbereich (30) umgeben sind, dass die Vielzahl einzelner durchlässiger Blendenbereiche(29) in mindestens zwei Gruppen unterteilbar ist, in denen sich die durchlässigen Blendenbereiche (29) in Form und/oder Größe unterscheiden, und
dass die durchlässigen Blendenbereiche (29) im Wege eines Material abtragenden Ätzvorganges gebildet sind.

5. Therapievorichtung nach einem der Ansprüche 1 bis 3,
dass die Strichplatte (33) eine Vielzahl für den Therapiestrahl (10) durchlässige Plattenbereiche (34) besitzt, die jeweils von einem für den Therapiestrahl (10) undurchlässigen Plattenbereich (35) zumindest teilweise umgeben sind,
dass die Vielzahl einzelner durchlässiger Plattenbereiche (34) in mindestens zwei Gruppen unterteilbar ist, in denen sich die durchlässigen Plattenbereiche (34) in Form und/oder Größe unterscheiden, und
dass die undurchlässigen Plattenbereiche (34) im Wege Druckverfahrens, vorzugsweise im Wege eines Diadurverfahrens oder Laserdruckverfahrens, gebildet sind.

6. Therapievorrichtung nach einem der Ansprüche 1 bis 5
**dadurch gekennzeichnet, dass** jeweils eine Lichtquelle (19) zur Erzeugung eines der wenigstens drei Ziellichtstrahlen (18) vorhanden ist, und
dass die das Blendenloch (20') der stenopäischen Blende (20) auf die okuläre Abbildungsebene (16) scharf abbildende optische Abbildungseinheit eine asphärische Linse (21) ist.

7. Therapievorrichtung nach den Ansprüchen 2 und 6,
**dadurch gekennzeichnet, dass** den wenigstens drei Ziellichtstrahlen (18) jeweils eine optische Achse zuordenbar ist, längs der jeweils die Lichtquelle (19), die stenopäische Blende (20) sowie die asphärische Linse (21) angeordnet sind.

8. Therapievorrichtung nach einem der Ansprüche 1 bis 7,
**dadurch gekennzeichnet, dass** die den Fixierlichtstrahl (24) auf die der okulären Abbildungsebene (16) in Strahlrichtung nachgelagerte Abbildungsebene abbildende Abbildungsoptik dem optischen Mittel zur Beeinflussung der dem Therapiestrahl zuordenbaren Strahlform entspricht.

9. Therapievorrichtung nach den Ansprüchen 2 und 8,
**dadurch gekennzeichnet, dass** die optische Umlenkeinheit (13) den Therapiestrahl (10) reflektiert und den Fixierlichtstrahl (24) transmittiert, und
dass längs der zweiten optischen Achse (14) zur optischen Umlenkeinheit (13) der asphärischen optischen Linse (15) gegenüberliegend eine Lichtquelle (22) zur Erzeugung des Fixierlichtstrahls (24) sowie eine stenopäische Blende (23) angeordnet sind.

## Claims

1. An ocular treatment device having a radiation source that emits UV light (4) as well as an optical imaging system located downstream from the radiation source (4) for imaging a therapy beam (10) emitted by the radiation source (4) in an ocular imaging plane (16), having an optical condenser unit (5) downstream from the radiation source (4), a diaphragm unit (11), an optical means for influencing a spatial energy distribution (12) that is oriented along the therapy beam cross section and can be assigned to the therapy beam as well as having an optical means for influencing a beam shape (15) that can be assigned to the therapy beam,
**characterized in that** the means for influencing the energy distribution that can be assigned to the therapy beam is a diaphragm produced by means of an etching technique, a so-called etching diaphragm (12) or a graticule (33) printed by means of printing technique,
the optical means for influencing the beam shape that can be attributed to the therapy beam comprises at least one aspherical optical lens (15),
at least three spatially separate target light beams (18) are arranged spatially around the therapy beam (10) directed at the ocular imaging plane (16), along each of which target light beams a stenopeic diaphragm (20) is introduced, its diaphragm hole (20') being imageable through an optical imaging unit (21) in the ocular imaging plane (16), so that the at least three target light beams (18) imaged in the ocular imaging plane (16) lie on a circular line (K), wherein the diameter of the circle is measured as being between 3 and 15 mm,
and
along the therapy beam (10) directed at the ocular imaging plane (16), a fixation light beam (24) oriented at the ocular imaging plane (16) is arranged, a stenopeic diaphragm (23) being introduced along the light beam and its diaphragm hole (23') being imageable over an imaging optical system in an imaging plane downstream from the ocular imaging plane (16) in the direction of the beam.

2. The treatment device according to Claim 1,
**characterized in that** the optical imaging system comprises
- the condenser unit (5), which images the therapy beam (10) emerging from the beam source (4) along a first optical axis (9) in a first intermediate image plane (6),
- an illumination optical system (7), which consists of at least two optical lenses (7.1, 7.2), imaging the first intermediate image plane (6) in a second intermediate image plane (8), and
- at least one aspherical optical lens (15), which images the second imaging plane (8) in the ocular imaging plane (16),
and
an optical deflecting unit (13) is introduced between the illumination optical system (7) and the at least one aspherical optical lens (15), deflecting the therapy beam (10) oriented along the first optical axis (9) into a second optical axis (14) directed at the ocular imaging plane (16).

3. The treatment device according to Claim 1 or 2,
**characterized in that** between the at least two optical lenses (7.1, 7.2) of the illumination optical system (7), an iris diaphragm (11) is arranged and between the illumination optical system (7) and the least one aspherical lens (15), the etching diaphragm (12) or the printed graticule (33) is arranged outside of the second intermediate image plane (8).

4. The treatment device according to any one of Claims 1 to 3,
**characterized in that** the etching diaphragm (12) has a plurality of diaphragm regions (29) that are permeable for the therapy beam (10), each being surrounded by a diaphragm region (30) that is opaque for the therapy beam (10),
the plurality of individual permeable diaphragm regions (29) can be subdivided into at least two groups, in which the permeable diaphragm regions (29) vary in shape and/or size, and
the permeable diaphragm regions (29) are formed by means of an etching process that removes material.

5. The treatment device according to any one of Claims 1 to 3,
**characterized in that** the graticule (33) has a plurality of plate regions (34) that are permeable for the therapy beam (10), each plate region being surrounded at least partially by a plate region (35) that is opaque for the therapy beam (10),
the plurality of individual permeable plate regions (34) can be subdivided into at least two groups in which the permeable plate regions (34) differ in shape and/or size, and the opaque plate regions (34) are formed by means of a printing method, preferably by means of a Diadur method or a laser printing method.

6. The treatment device according to any one of Claims 1 to 5,
**characterized in that** a light source (19) is present for generating one of the at least three target light beams (18), and
the optical imaging unit which sharply images the diaphragm hole (20') of the stenopeic diaphragm (20) on the ocular imaging plane (16) is an aspherical lens (21).

7. The treatment device according to Claims 2 and 6,
**characterized in that** an optical axis can be assigned to the at least three target light beams (18), the light source (19), the stenopeic diaphragm (20) and the aspherical lens (21) being arranged along this optical axis.

8. The treatment device according to any one of Claims 1 to 7,
**characterized in that** the imaging optical system that images the fixation light beam (24) on the imaging plane downstream from the ocular imaging plane (16) in the direction of the beam corresponds to the optical means for influencing the beam form that can be assigned to the therapy beam.

9. The treatment device according to Claims 2 and 8,
**characterized in that** the optical deflecting unit (13) reflects the therapy beam (10) and transmits the fixation light beam (24),
a light source (22) for generating the fixation light beam (24) as well as a stenopeic diaphragm (23) being arranged along the second optical axis (14) to the optical deflecting unit (13) of the aspherical optical lens (15).

## Revendications

1. Dispositif de thérapie oculaire comportant une source de rayonnement (4) émettant une lumière UV ainsi qu'un système d'imagerie optique disposé après la source de rayonnement (4) pour l'imagerie d'un rayon de thérapie (10) sortant de la source de rayonnement (4) dans un plan d'imagerie oculaire (16), comportant une unité de condensateur optique (5) disposée après la source de rayonnement (4), une unité de diaphragme (11), un moyen optique pour influencer une distribution d'énergie (12) spatiale orientée longitudinalement à la section transversale de rayon de thérapie, pouvant être coordonné au rayon de thérapie ainsi qu'un moyen optique pour influencer une forme de rayon (15) pouvant être coordonnée au rayon de thérapie,
**caractérisé en ce que** le moyen pour influencer la distribution d'énergie pouvant être coordonnée au rayon de thérapie est un diaphragme usiné au moyen d'une technique de gravure, appelée un diaphragme de gravure (12) ou un réticule (33) imprimé au moyen d'une technique d'impression,
**en ce que** le moyen optique pour influencer la forme de rayon pouvant être coordonnée au rayon de thérapie comprend au moins une lentille optique oculaire (15), **en ce que** spatialement autour du rayon de thérapie (10) orienté sur le plan d'imagerie oculaire (16) au moins trois rayons de lumière cible (18) séparés spatialement sont disposés, le long desquels respectivement un diaphragme sténopéique (20) est monté, dont le trou de diaphragme (20') peut être imagé respectivement par l'intermédiaire d'une unité d'imagerie optique (21) dans le plan d'imagerie oculaire (16), de sorte que les au moins trois rayons de lumière cible (18), imagés dans le plan d'imagerie oculaire (16) soient situés en étant également répartis sur une ligne de cercle (K), dont le diamètre circulaire mesure entre 3 et 15 mm, et **en ce que** le long du rayon de thérapie (10) orienté sur le plan d'imagerie oculaire (16) est disposé un rayon de lumière de fixation (24) orienté sur le plan d'imagerie oculaire (16), le long duquel un diaphragme sténopéique (23) est monté, dont le trou de diaphragme (23') peut être imagé par l'intermédiaire d'une optique d'imagerie dans un des plan d'imagerie oculaire (16) dans le plan d'imagerie en aval dans la direction de rayon.

2. Dispositif de thérapie selon la revendication 1, **caractérisé en ce que** le système d'imagerie optique comprend
- l'unité de condensateur (5), qui image le rayon de thérapie (10) sortant de la source de rayonnement (4) le long d'un premier axe optique (9) dans un premier plan d'image intermédiaire (6),
- une optique d'illumination (7) constituée d'au moins deux lentilles optiques (7.1, 7.2), qui image le premier plan d'image intermédiaire (6) dans un deuxième plan d'image intermédiaire (8), ainsi que
- au moins une lentille (15) optique asphérique, qui image le deuxième plan d'imagerie (8) dans le plan d'imagerie oculaire (16),
**en ce que** entre l'optique d'illumination (7) et au moins une lentille optique asphérique (15) est montée une unité de déviation optique (13), qui dévie le rayon de thérapie (10) orienté le long du premier axe optique (9) dans un deuxième axe optique (14) orienté sur le plan d'imagerie oculaire (16).

3. Dispositif de thérapie selon la revendication 1 ou 2, **caractérisé en ce que** entre au moins deux lentilles optiques (7.1, 7.2) de l'optique d'illumination (7) un diaphragme d'iris (11 est disposé et entre l'optique d'illumination (7) et au moins une lentille asphérique (15) le diaphragme de gravure (12) ou le réticule imprimé (33) sont disposés à l'extérieur du deuxième plan d'image intermédiaire (8).

4. Dispositif de thérapie selon une des revendications 1 à 3, **caractérisé en ce que** le diaphragme de gravure (12) possède une pluralité de zones de diaphragme (29) perméables au rayon de thérapie (10), qui sont respectivement entourées par une zone de diaphragme (30) imperméable au rayon de thérapie (10), **en ce que** la pluralité de régions de diaphragme (29) individuelles perméables peuvent être divisées en au moins deux groupes, parmi lesquels les zones de diaphragme (29) perméables se différencient en forme et/ou taille, et **en ce que** les zones de diaphragme perméables (29) sont formées au cours d'un processus de gravure par enlèvement de matière.

5. Dispositif de thérapie selon une des revendications 1 à 3, **caractérisé en ce que** le réticule (33) possède une pluralité de zones d plaques (34) transparentes au rayon de thérapie (10), qui sont respectivement entourées au moins partiellement par une zone de plaque (35) imperméable au rayon de thérapie (10), **en ce que** la pluralité de zones de plaques (34) perméables individuelles peuvent être divisées en au moins deux groupes, dans lesquels les zones de plaques perméables (34) se différencient en forme et/ou taille et **en ce que** les zones de plaques imperméables (34) sont formées au cours d'un procédé d'impression, de préférence au cours d'un procédé Diadur ou un procédé d'impression laser.

6. Dispositif de thérapie selon une des revendications 1 à 5, **caractérisé en ce que** respectivement une source de lumière (19) pour produire un des au moins trois rayons de lumière cible (18) est prévu, et **en ce que** l'unité d'imagerie optique imageant avec une haute résolution le trou de diaphragme (20') du diaphragme sténopéique (20) sur le plan d'imagerie oculaire (16) est une lentille asphérique (21).

7. Dispositif de thérapie selon les revendications 2 et 6, **caractérisé en ce que** les au moins trois rayons de lumière cible (18) peuvent être coordonnés respectivement à un axe optique, le long duquel respectivement la source de lumière (19), le diaphragme sténopéique (20) ainsi que la lentille asphérique (21) sont disposés.

8. Dispositif de thérapie selon une des revendications 1 à 7, **caractérisé en ce que** l'optique d'imagerie imageant le rayon de lumière de fixation (24) sur le plan d'imagerie en aval du plan d'imagerie oculaire (16) dans la direction de rayon correspond au moyen optique pour influencer la forme de rayon pouvant être coordonnée au rayon de thérapie.

9. Dispositif de thérapie selon les revendications 2 et 8, **caractérisé en ce que** l'unité de déviation optique (13) réfléchit le rayon de thérapie (10) et transmet le rayon de lumière de fixation (24) et **en ce que** le long du deuxième axe optique (14) en vis-à-vis de l'unité de déviation optique (13)de la lentille optique asphérique (15) une source de lumière (22) pour produire le rayon de lumière de fixation (24) ainsi qu'une diaphragme sténopéique (23) sont disposés.
